# EUROPEAN PATENT APPLICATION

(11) **EP 3 872 732 A1**
(43) Date of publication of application: **01.09.2021**
(21) Application number: 19876707.1
(22) Date of filing: 24.10.2019
(51) Int. Cl.: G06Q 20/38, G06Q 20/06, G06Q 20/40, G16B 99/00

(54) **COLORED COIN HAVING INDIVIDUAL GENE PHENOTYPE APPLIED THERETO**

(30) Priority: 24.10.2018 KR 20180127634
(71) Applicant: BA Genomics Inc., Seoul 06624 (KR)
(72) Inventor: KWON, Chang Hyuk, Anyang-si, Gyeonggi-do 14051 (KR); OH, Tae Min, Seongnam-si, Gyeonggi-do 13581 (KR); SHIN, Shang Cheol, Incheon 22014 (KR)
(74) Representative: Dehns
(86) International application number: PCT/KR2019/014031
(87) International publication number: WO 2020/085806

(57) **Abstract**

The present invention relates to a colored coin having an individual gene phenotype applied thereto. Particularly, a colored coin manufactured by a method according to the present invention and having an individual gene phenotype applied thereto such that an individual can be identified, cannot only be circulated freely as a Bitcoin block or colored coin metadata, but also is useful in various fields, such as gene-based character fabrication and gaming.

## Description

### BACKGROUND OF THE INVENTION

### Field of the invention

The present disclosure relates to a colored coin having an individual gene phenotype applied thereto.

### Related Art

Blockchain refers to a data management technology that records continuously increasing data in units of blocks and allows each blockchain node to constitute a peer-to-peer (P2P) network to manage the blocks in a chain-like data structure, or blockchain refers to the data structure itself. In this case, blockchain data of the chain-like data structure is managed in the form of a distributed ledger at each individual node, without a central system.

Each individual blockchain node of a blockchain network manages blocks in such a data structure. Here, each block is recorded with a hash value of a previous block, so that the previous block can be referred to by the hash value. Therefore, as more blocks are added, it becomes difficult to forge transaction data recorded in the block, and the transaction data recorded in each block is improved in reliability.

Today, with the merits of the foregoing blockchain technology, there have been proposed various systems for providing a transaction service of a cryptocurrency issued and circulated through a blockchain. The Bitcoin system is a representative example of cryptocurrency transaction systems. The Bitcoin system performs unspent transaction output (UTXO) verification for each transaction, in order to prevent double spending and provide a safe transaction service.

Open Asset Protocol is one of the most common derivative technologies for increasing the usefulness of cryptocurrency exchanged through a blockchain. Open Asset Protocol is an asset issuing technology that defines and issues various types of user-defined currencies based on a cryptocurrency issued on a blockchain.

Colored coins, which are a type of open asset protocol, use Bitcoin's scripting language to store small amounts of metadata on the blockchain, which can be used to represent intellectual assets as irreversible and unforgeable tokens.

### SUMMARY OF THE INVENTION

The present disclosure provides a colored coin having an individual gene phenotype applied thereto.

To achieve the above purpose, the present disclosure provides the method of generating colored coin. The method comprises 1.1) a method of selecting a gene phenotype set made up of many lists that are not sensitive and do not involve clinical markers that can identify an individual, such as hair color, hair type, eye color, skin color, sex, and eye color; 1.2) a method of inserting a gene phenotype set into a colored coin, with the size minimized to about 200 bytes; 1.3) a method of inserting a gene phenotype and a hashed value of the entire gene information side by side into a colored coin; 1.4) every method of transaction in which a gene phenotype set is put into a blockchain platform, i.e., Ethereum or EOS; and 1.5) a method of generating a key by arranging genotypes in a specific order in a phenotype set and using the same as a seed value for a hash key.

A colored coin manufactured by a method according to the present disclosure, and having an individual gene phenotype applied thereto such that an individual can be identified, cannot only be circulated freely as a Bitcoin block or colored coin metadata, but also is useful in various fields, such as gene-based character fabrication and gaming.

### BRIEF DESCRIPTION OF THE DRAWINGS

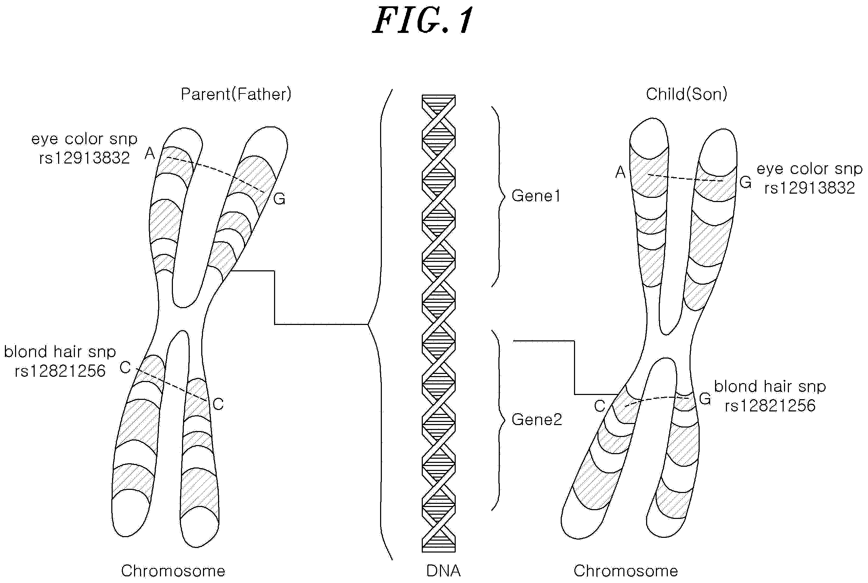

FIG. 1 is a pattern diagram showing the definition of an individual's gene phenotype set and the selection of the number thereof.

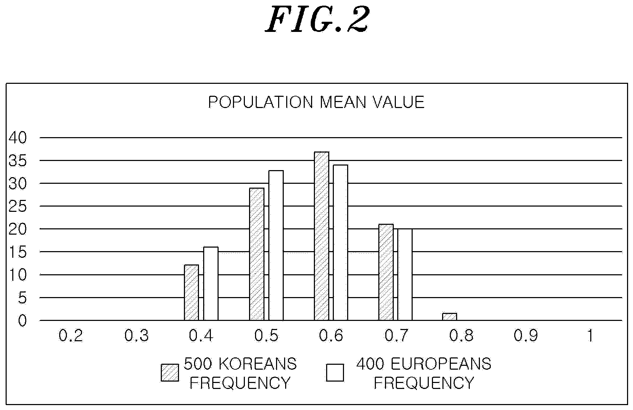

FIG. 2 is a result graph showing the population mean values of frequency of 100 phenotype sets in Koreans and Europeans.
FIG. 3 is a pattern diagram showing a process in which each individual's phenotypes are inserted into a colored coin by inserting them into a Bitcoin block.
FIG. 4 is a pattern diagram showing a process in which each individual's phenotypes are inserted into a colored coin by inserting a phenotype set and a hash value together into a Bitcoin block.
FIG. 5 is a pattern diagram showing a process in which each individual's phenotypes are inserted into a colored coin by generating a key.

### DESCRIPTION OF EXEMPLARY EMBODIMENTS

Hereinafter, the present disclosure will be described in details.

The present disclosure provides a colored coin, which is created by using: 1.1) a method of selecting a gene phenotype set made up of many lists that are not sensitive and do not involve clinical markers that can identify an individual, such as hair color, hair type, eye color, skin color, sex, and eye color; 1.2) a method of inserting a gene phenotype set into a colored coin, with the size minimized to about 200 bytes; 1.3) a method of inserting a gene phenotype set and a hashed value of the entire gene information side by side into a colored coin; 1.4) every method of transaction in which a gene phenotype set is put into a blockchain platform, i.e., Ethereum or EOS; and 1.5) a method of generating a key by arranging genotypes in a specific order in a phenotype set and using the same as a seed value for a hash key.

In the colored coin created according to the present disclosure, in the step 1.1), a set for identifying an individual may be selected by using a non-specific marker list of 100 or more markers, by taking many repeat markers of the same race into consideration (see Embodiment 1). Also, in the step 1.1), some or all of vcf, whole exome vcf, and whole genome vcf lists may be selected as only one phenotype set by using a genotyping array chip and an exome chip to represent at least 100,000 markers. Furthermore, in the step 1.1), a phenotype set may be made using 10,000 or more marker lists generally used for ancestry identification.

In the colored coin created according to the present disclosure, in the step 1.2), the only one phenotype set, which takes up about 200 bytes out of the block size of 1 MB to 32 Mb in most colored coins including Bitcoin and Bitcoin cash, may be inserted into a data region or inserted in the form of metadata userData (see Embodiment 2).

In the colored coin created according to the present disclosure, in the step 1.5), 2-byte alphabet letters are arranged in a predetermined order and used as only one seed value of a phenotype set of genotypes to generate a hash key. (See Embodiment 3).

A colored coin created according to the present disclosure may be used to manufacture, advertise, or sell a character using an individual's genes by using the individual's phenotype set. Also, the colored coin may be used to manufacture, advertise, or sell an individual's gene-based gene game by using the individual's phenotype set.

Hereinafter, the present disclosure will be described in details by the following embodiments. However, the following embodiment are only for illustration, but not intended to limit the present disclosure. Any embodiments having substantially the same structure and achieving the same operational effects as the technical spirit described in claims of the present disclosure are encompassed in the technical field of the present disclosure.

### Embodiment 1. Selection of Phenotype Set

A phenotype set was selected from an individual's gene information that is related to the person's appearance but not sensitive, such as hair color, eye color, skin color, drinking habits, sexual behavior, birth rate, longevity, obesity, birth weight, muscle content, and malnutrition. The following Tables 1 and 2 show a phenotype set of skin color, eye color, and hair color selected from gene information of Koreans and Europeans.

**[Table 1]**

| Type | rsId | Korean1 Genotype | Population Frequency | European1 Genotype | Population Frequency |
|---|---|---|---|---|---|
| Skin | rs1488799 | TT | 0.90 | CC | 0.50 |
| | rs1800414 | CC | 0.30 | AA | 1.00 |
| | rs2733832 | CC | 0.90 | TT | 0.40 |
| Eye | rs105362 | GG | 0,50 | GG | 0.50 |
| | rs12913823 | CC | 0.90 | CT | 0.40 |
| | rs4911414 | TG | 0.30 | GG | 0.50 |
| | rs4911442 | AA | 0.90 | AA | 0.80 |
| | rs6058017 | AA | 0.50 | AG | 040 |
| Hair | rs12821256 | TT | 1.00 | TT | 0.70 |
| | rs1540771 | GG | 0.50 | AG | 0.50 |
| | rs35264875 | AA | 1.00 | AA | 0.70 |
| | rs3829241 | AG | 0.40 | AG | 0.50 |
| | rs12896399 | GG | 0.30 | GT | 0.50 |
| | rs12203592 | CC | 1.00 | CC | 0.70 |
| ... | ...... | | | | |
| [Population Mean Val] Average | | | 0.66 | | 0.57 |

Also, 900 Koreans and Europeans were randomly sampled for final selected 100 phenotype lists. As shown in Table 2 below, the results showed that 2.037E-10 alone represented the global population at values 0.8 or under.

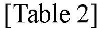

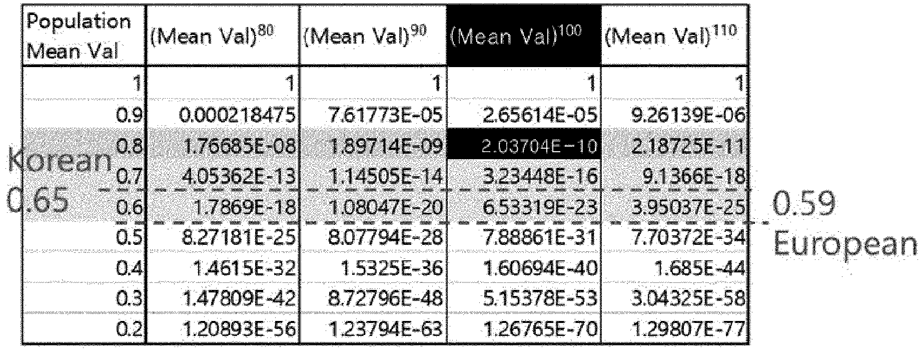

### Embodiment 2. Insertion into Colored Coin

### 2-1. Insertion of Phenotype Set into Bitcoin Block

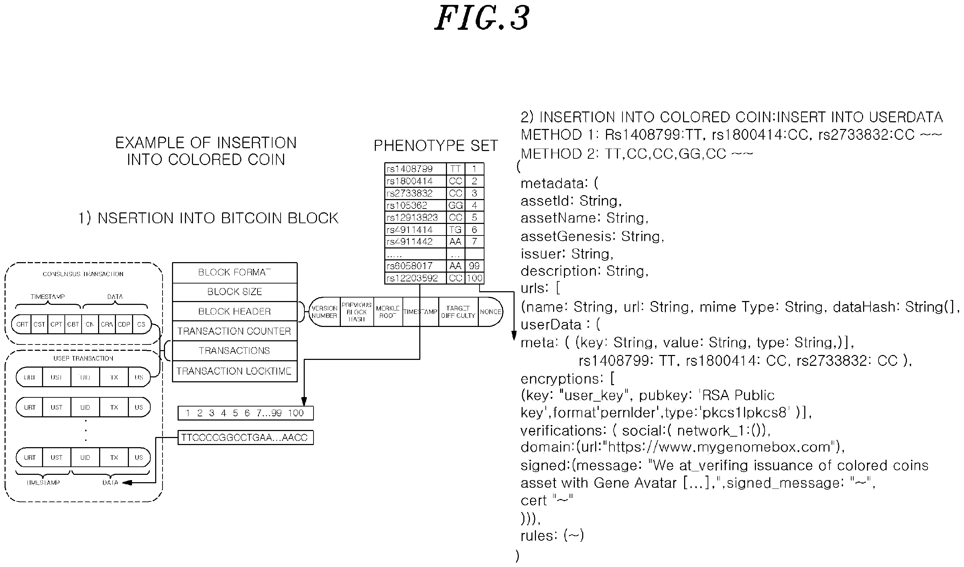

A phenotype set selected in Embodiment 1 was inserted into a Bitcoin block by the method depicted in the pattern diagram of FIG. 3.

### 2-2. Insertion of Phenotype Set and Hash Value

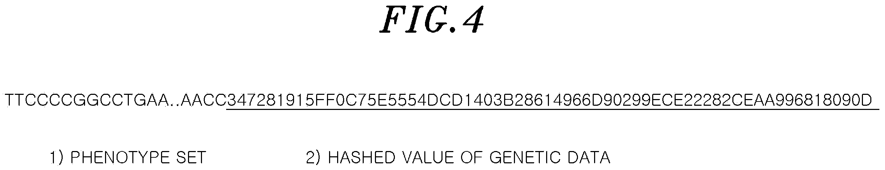

As shown in FIG. 4, a phenotype set selected in Embodiment 1 was inserted, along with a hash value (e.g., a hashed value of genetic data), into a Bitcoin block.

### 2-3. Generation of Key

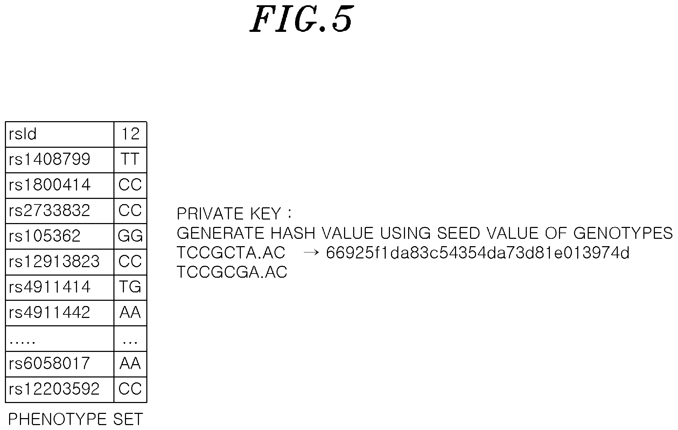

As shown in FIG. 5, a phenotype set selected in Embodiment 1 was used to generate a private key. In this case, the private key was calculated as a hash value by using a seed value of the genotypes of the selected phenotype.

## Claims

1. A colored coin, which is created by using:
1.1) a method of selecting a gene phenotype set made up of many lists that are not sensitive and do not involve clinical markers that can identify an individual, such as hair color, hair type, eye color, skin color, sex, and eye color;
1.2) a method of inserting a gene phenotype set into a colored coin, with the size minimized to about 200 bytes;
1.3) a method of inserting a gene phenotype and a hashed value of the entire gene information side by side into a colored coin (see Embodiment 2);
1.4) every method of transaction in which a gene phenotype set is put into a blockchain platform, i.e., Ethereum or EOS; and
1.5) a method of generating a key by arranging genotypes in a specific order in a phenotype set and using the same as a seed value for a hash key.

2. The colored coin of claim 1, wherein, in the step 1.1), a set for identifying an individual is selected by using a non-specific marker list of 100 or more markers, by taking many repeat markers of the same race into consideration.

3. The colored coin of claim 1, wherein, in the step 1.1), some or all of vcf, whole exome vcf, and whole genome vcf lists are selected as only one phenotype set by using a genotyping array chip and an exome chip to represent at least 100,000 markers.

4. The colored coin of claim 1, wherein, in the step 1.1), a phenotype set is made using 10,000 or more marker lists generally used for ancestry identification.

5. The colored coin of claim 1, wherein, in the step 1.2), the only one phenotype set, which takes up about 200 bytes out of the block size of 1MB to 32Mb in most colored coins including Bitcoin and Bitcoin cash, is inserted into a data region or inserted in the form of metadata userData.

6. The colored coin of claim 1, wherein, in the step 1.5), 2-byte alphabet letters are arranged in a predetermined order and used as only one seed value of a phenotype set of genotypes to generate a hash key.
